**Europäisches Patentamt**

**European Patent Office**

**Office européen des brevets**

(19)

(11) Veröffentlichungsnummer: **0 112 544**
**A1**

(12) # EUROPÄISCHE PATENTANMELDUNG

(21) Anmeldenummer: **83112745.1**

(22) Anmeldetag: **17.12.83**

(51) Int. Cl.³: **C 07 C 19/045**, C 07 C 17/02,
C 07 C 17/38

(30) Priorität: **24.12.82 DE 3247988**

(43) Veröffentlichungstag der Anmeldung: **04.07.84**
**Patentblatt 84/27**

(84) Benannte Vertragsstaaten: **BE CH DE FR GB IT LI NL SE**

(71) Anmelder: **HOECHST AKTIENGESELLSCHAFT,**
**Postfach 80 03 20, D-6230 Frankfurt am Main 80 (DE)**

(72) Erfinder: **Hundeck, Joachim, Dr., Argelander**
**Strasse 135, D-5300 Bonn (DE)**
Erfinder: **Scholz, Harald, Dr., Am Beissel 8,**
**D-5042 Erftstadt (DE)**
Erfinder: **Hennen, Hans, Kendenicher Strasse 86,**
**D-5030 Hürth (DE)**

(54) **Verfahren zur Herstellung von 1,2-Dichlorethan.**

(57) Die Erfindung betrifft ein Verfahren zur Herstellung von 1,2-Dichlorethan durch Reaktion von Ethylen mit Chlor in einem Lösungsmittel in Gegenwart eines Katalysatorgemisches bestehend aus wasserfreiem Eisen-III-chlorid und einer Stickstoffbase bzw. einem Salz dieser Base bei einer Temperatur unterhalb des Siedepunktes des 1,2-Dichlorethans und Abtrennen des 1,2-Dichlorethans aus dem Chlorierungsgemisch, welches dadurch gekennzeichnet ist, daß man
a) das Chlorierungsgemisch in eine Destillationskolonne fördert, aus dem Gemisch das 1,2-Dichlorethan bis zur Ausscheidung des Katalysators aus dem flüssigen Sumpfprodukt abdestilliert, aus dem Sumpfprodukt den ausgeschiedenen Katalysator abtrennt und letzteren erneut zur Umsetzung von Ethylen mit Chlor verwendet oder daß man
b) das Chlorierungsgemisch über ein Adsorptionsmittel leitet, den im Gemisch enthaltenen Katalysator an dem Adsorptionsmittel adsorbiert, aus dem anfallenden Filtrat 1,2-Dichlorethan durch Destillation abtrennt und den Katalysator aus dem Adsorptionsmittel zurückgewinnt oder daß man
c) entsprechend a) verfährt, jedoch bei geringen Katalysatorgehalten das katalysatorhaltige Sumpfprodukt verwirft.

Verfahren zur Herstellung von 1,2-Dichlorethan

Die Herstellung von 1,2-Dichlorethan durch Reaktion von Ethylen mit Chlor in 1,2-Dichlorethan als Lösungsmittel und Reaktionsmedium ist bekannt. Zur Beschleunigung der Chloraddition verwendet man als Katalysator neben den Chloriden der Elemente der IV. bis VI. Gruppe des Periodensystems vor allem wasserfreies Eisen-III-chlorid, gegebenenfalls in Gegenwart von Sauerstoff, da Eisen-III-chlorid leicht zugänglich und preisgünstig ist. Als hauptsächliches Nebenprodukt fällt bei dieser Reaktion 1,1,2-Trichlorethan durch Substitution von Dichlorethan an.

Die Chloraddition an Ethylen wird in der Technik sowohl bei Reaktionstemperaturen um 90°C, wobei das Reaktionsprodukt unter Ausnutzung der freigesetzten Reaktionswärme aus dem Reaktionsgemisch abdestilliert wird, als auch bei tieferen Temperaturen von 30 - 60°C, wie in Ullmanns Encyklopädie der technischen Chemie, Bd. 9, 4. Auflage 1975, Seite 427 beschrieben, durchgeführt. Die Reaktionswärme kann in letzterem Falle nicht für die Rektifikation des Rohdichlorethans herangezogen werden sondern muß, wie beispielsweise in der DE-OS 19 05 517 ausgeführt, durch Umpumpen des Reaktionsmediums über Wärmetauscher abgeführt werden.

2

Das bei tieferen Temperaturen hergestellte rohe, katalysatorhaltige Dichlorethan wurde bisher im allgemeinen aus dem Reaktionsgefäß abgezogen und zur Entfernung des Katalysators und des im Rohprodukt enthaltenen Chlorwasserstoffes mit Wasser bzw. wäßrigen Alkalilösungen gewaschen, das Dichlorethan von der wäßrigen Schicht abgetrennt und anschließend in bekannter Weise destillativ aufgearbeitet.

Die Verwendung von $FeCl_3$ als Katalysator bei der Additionschlorierung von Ethylen ist mit gewissen Nachteilen verbunden. So wirkt $FeCl_3$ in Gegenwart von Wasser gegenüber dem metallischen Werkstoffen von Reaktoren, Kolonnen oder Wärmeaustauschern, soweit diese mit dem Katalysator in Berührung kommen, korrosiv. Das zur Chlorierung normalerweise verwendete Chlor mit technischem Reinheitsgrad enthält stets Spuren von Feuchtigkeit, außerdem entsteht immer Chlorwasserstoff aus unerwünschten Nebenreaktionen.

Nach dem Verfahren der DE-OS 31 48 450 kann die durch $FeCl_3$ als Katalysator bei der Herstellung von 1,2-Dichlorethan verursachte Korrosion in nicht korrosionsfesten Reaktoren erheblich vermindert werden, wenn man den $FeCl_3$-Katalysator mit bestimmten Zusätzen beaufschlagt. Darüberhinaus wurde festgestellt,. daß diese Zusätze sich auch vorteilhaft auf die Nebenproduktbildung, welche dadurch verringert wird, auswirken.

Gegenstand der DE-OS 31 48 450 ist ein Katalysatorgemisch, bestehend aus wasserfreiem Eisen-III-chlorid sowie einer weiteren Gemischkomponente zur Herstellung von 1,2-Dichlorethan durch Reaktion von Ethylen mit Chlor in einem Lösungsmittel bei Normal- oder Überdruck, welches dadurch gekennzeichnet ist, daß die weitere Gemischkomponente eine in Bezug auf die Eisen-III-chlorid-Menge etwa äquivalente Menge einer Stickstoffbase oder ein Salz dieser Base ist.

Die Stickstoffbase kann $NH_3$, ein primäres, sekundäres oder tertiäres Alkyl-, Aralkyl-, Aryl- oder alicyclisches Amin oder ein Polyamin sein. Das Salz der Stickstoffbase ist vorzugsweise ein Halogensalz, beispielsweise Ammoniumchlorid.

Das in der DE-OS 31 48 45o beschriebene Katalysatorgemisch eignet sich besonders für die Durchführung der Chlorierung von Ethylen bei erhöhter Temperatur, wobei die Reaktionswärme der Ethylenchlorierung genutzt werden kann derart, daß der produzierte Anteil des 1,2-Dichlorethans aus dem Reaktionsgemisch dampfförmig abgetrieben wird. Der Katalysator bleibt dabei im Reaktionsgemisch, so daß allenfalls geringfügige Katalysatorverluste von Zeit zu Zeit ausgeglichen werden müssen.

Im Gegensatz dazu wird bei Chlorierungsverfahren, die unterhalb des Siedebereiches von 1,2-Dichlorethan betrieben werden, der Katalysator aus dem gebildeten rohen Dichlorethan beispielsweise mit Wasser ausgewaschen, wobei der Katalysator im Waschwasser gelöst ist, so daß der hierbei eintretende Katalysatorverlust durch Zusatz von frischem Katalysator ausgeglichen werden muß.

Da die beim Verfahren der DE-OS 31 48 45o eingesetzten katalysatorbildenden Komponenten in Form von wasserfreiem $FeCl_3$ und einer Stickstoffbase im Chlorierungsansatz zu einem auch in geringen Mengen hochwirksamen Katalysator reagieren, hat es sich als vorteilhaft erwiesen, diesen Katalysator bei der Aufarbeitung des Chlorierungsgemisches in Abhängigkeit von seiner Konzentration im Chlorierungsgemisch zurückzugewinnen, um ihn erneut für die Reaktion des Ethylens mit Chlor verwenden zu können. Geeignete Wege zur Aufarbeitung des obigen Katalysator enthaltenden Chlorierungsgemisches werden

4

durch das erfindungsgemäße Verfahren vermittelt.

Gegenstand der Erfindung ist somit ein Verfahren zur Herstellung von 1,2-Dichlorethan durch Reaktion von Ethylen mit Chlor in einem Lösungsmittel in Gegenwart eines Katalysatorgemisches bestehend aus wasserfreiem Eisen-III-chlorid und einer Stickstoffbase bzw. einem Salz dieser Base sowie gegebenenfalls eines Inhibitors zur Verhinderung der Nebenproduktbildung bei einer Temperatur unterhalb des Siedepunktes des 1,2-Dichlorethans von etwa 20 - 100°C und Normal- oder Überdruck und Abtrennen des 1,2-Dichlorethans aus dem Chlorierungsgemisch, welches dadurch gekennzeichnet ist, daß man

a) das Chlorierungsgemisch in eine Destillationskolonne fördert, aus dem Gemisch das 1,2-Dichlorethan bis zur Ausscheidung des Katalysators aus dem flüssigen Sumpfprodukt abdestilliert, aus dem Sumpfprodukt den ausgeschiedenen Katalysator abtrennt und letzteren erneut zur Umsetzung von Ethylen mit Chlor verwendet oder daß man

b) das Chlorierungsgemisch über ein Adsorptionsmittel leitet, den im Gemisch enthaltenen Katalysator an dem Adsorptionsmittel adsorbiert, aus dem anfallenden Filtrat 1,2-Dichlorethan durch Destillation abtrennt und den Katalysator aus dem Adsorptionsmittel durch Eluierung mit einem Lösungsmittel zurückgewinnt oder daß man

c) entsprechend a) verfährt, jedoch bei geringen Katalysatorgehalten das katalysatorhaltige Sumpfprodukt verwirft.

In Ausübung des Verfahrens der Erfindung wurde festgestellt, daß die Konzentration des $FeCl_3$ im Reaktionsansatz auf

0,001 bis etwa 0,5 Gew%, insbesondere 0,002 bis 0,01 Gew%, bezogen auf die Menge des Lösungsmittels, zu bemessen ist, während die Menge der Stickstoffbase bzw. deren Salz äquivalent zur $FeCl_3$-Menge sein soll.

Als Lösungsmittel hat sich wie bei den entsprechenden bekannten Verfahren zur Herstellung von 1,2-Dichlorethan, auch in vorliegendem Falle das 1,2-Dichlorethan selbst bewährt.

Nach einer bevorzugten Ausführungsform der Erfindung, betreffend die Behandlung des Chlorierungsgemsiches mit einem Adsorptionsmittel, hat sich der Einsatz von Aktivkohle, basischen Ionenaustauschern oder $SiO_2$ bzw. Aluminiumoxid als zweckmäßig erwiesen. Zur Eluierung des Katalysators vom Adsorptionsmittel empfiehlt sich die Verwendung von 1,2-Dichlorethan oder anderen leichtflüchtigen chlorierten Kohlenwasserstoffen oder Salzsäure.

Die alternative Ausführungsform des erfindungsgemäßen Verfahrens, bestehend darin, auf die Isolierung des Katalysators zu verzichten und das katalysatorhaltige Sumpfprodukt zu verwerfen, ist dann wirtschaftlich von Vorteil, wenn das Sumpfprodukt einen Katalysatorgehalt von höchstens etwa 0,001 Gew%, bezogen auf die flüssigen Anteile des Sumpfproduktes, aufweist und deshalb die Abtrennung des Katalysators Schwierigkeiten bereiten würde.

Der beim Verfahren der Erfindung angewandte Katalysator ist als technisch fortschrittlich zu bezeichnen, da durch ihn die Nebenproduktbildung mit Ausnahme geringer Mengen an 1,1,2-Trichlorethan und einer entsprechend geringen Menge von Chlorwasserstoff unterdrückt bzw. weitgehend vermieden wird. Außerdem bleibt auch bei längerer Reaktionsdauer die Reaktionslösung hell, wenn die dem Chlorierungsansatz zugefügte Stickstoffbase beispielsweise Ammoniak ist. Ein im Verlauf der Reaktion eventuell dunkel gefärbtes Reaktionsgemisch hellt sich im Ausmaß

6

der Ammoniak-Zugabe nach kurzer Zeit wieder auf. Schließlich ist festzustellen, daß beim Verfahren der Erfindung der Umsatz bei hoher Raum/Zeit-Ausbeute nahezu quantitativ ist. Dieses Ergebnis wird auch bei Katalysatorkonzentrationen von weniger als 0,003 Gew%, bezogen auf $FeCl_3$, erzielt.

Die gemäß Erfindung erteilte technische Lehre zur Aufarbeitung des Chlorierungsgemisches war nicht naheliegend, da ihr die Erkenntnis vorausgehen mußte, daß das zur Aufarbeitung bestimmte Chlorierungsgemisch nicht mehr die eingesetzten Katalysatorkomponenten in Form von $FeCl_3$ und der Stickstoffbase enthält, sondern daß die katalytisch wirksame Substanz das Reaktionsprodukt aus $FeCl_3$ und der Stickstoffbase ist, welches hinsichtlich seiner katalytischen Aktivität die des $FeCl_3$ bei weitem übertrifft. Insofern erwies es sich als sinnvoll, diesen Katalysator, im Gegensatz zu $FeCl_3$ bei den herkömmlichen Verfahren, zurück zu gewinnen.

Das Verfahren der Erfindung kann beispielsweise in dem in der DE-OS 24 27 045 beschriebenen Schlaufenreaktor oder auch in jedem anderen geeigneten Reaktor durchgeführt werden.

Beispiel 1

Ein zylindrischer Reaktor, dessen Volumen ca. 25 m$^3$ betrug, wurde mit 20.000 Litern 1,2-Dichlorethan beschickt und die Reaktorfüllung mit 170 mg Eisen-III-chlorid pro kg 1,2-Dichlorethan versetzt. Außerdem wurden dieser Lösung ca. 600 l Ammoniak beigefügt. Bei einem Druck am Kopf des Reaktors von ca. 1,3 bar und einer Temperatur des Reaktionsgemisches von ca. 40°C wurden in den Reaktor stündlich 1.008 kg Ethylen und 2.540 kg Chlorgas, welches

7

zusätzlich etwa 4 Vol% inerte Gase enthielt, sowie 8 m³ Luft eingeleitet. Zusätzlich wurde der Chlorierungsansatz mit ca. 80 l Ammoniak und 170 mg $FeCl_3$ pro Stunde versetzt.

Aus dem Reaktor wurden ca. 3.542 kg/h rohes Dichlorethan abgezogen und dem Sumpf einer Distillationskolonne zugeführt. Das zugeführte Rohprodukt besaß folgende Zusammensetzung:

| | |
|---|---|
| $C_2H_4$ | 0,0069 Gew% |
| $C_2H_5Cl$ | 0,0081 Gew% |
| 1,2-EDC | 99,8 Gew% |
| 1,1,2,ETC | 0,15 Gew% |

(EDC = Dichlorethan, ETC = Trichlorethan)

Die Kolonne wurde mit einem mäßigen Unterdruck (etwa 0,7 bar absolut) am Kopf der Kolonne betrieben. Aus dem Reaktionsgemisch wurden 95 % des erwünschten Reaktionsproduktes abdestilliert, wobei das gereinigte 1,2-Dichlorethan folgende Zusammensetzung aufwies:

| | |
|---|---|
| $C_2H_5Cl$ | 0,01 Gew% |
| 1,2-EDC | 99,98 Gew% |
| 1,1,2-ETC | 0,01 Gew% |

Der Sumpf der Kolonne wurde nach Abkühlung über ein Filter geleitet und filtriert. Das Filtrat wurde zur weiteren Aufarbeitung einer Vakuum-Kolonne zugeführt. Der abgetrennte Katalysator wurde dem Filter unter Ausschluß von Luftfeuchtigkeit entnommen und einem Lösebehälter zugeführt, welcher mit 1,2-Dichlorethan beschickt war. Im weiteren Verlauf des Chlorierungsprozesses wurde die für die Reaktion nötige Katalysatormenge aus dem zurückgewonnenen Anteil zugegeben, während die Eisen-III-chlorid- und Ammoniak-Zufuhr abgestellt werden konnte.

8

Beispiel 2

Von dem gemäß Beispiel 1 erhaltenen Rohprodukt wurden stündlich ca. 1,2 kg über eine Kolonne geleitet, die auf einer Länge von 850 mm eine Schüttung von 335 g Aktivkohle enthielt. Im Ablauf des Absorbers konnte im Verlauf von 6 Tagen kein Eisen nachgewiesen werden. Aus dem erhaltenen Filtrat wurde das 1,2-Dichlorethan durch Destillation abgetrennt. Die Aktivkohle wurde mit verdünnter Salzsäure regeneriert.

Beispiel 3

Es wurde ein, nach der Verfahrensweise gemäß Beispiel 1 hergestelltes Reaktionsgemisch, mit einem Gehalt von 0,275 Gew% $FeCl_3$ und einer äquivalenten Menge Ammoniak zur Abtrennung des Katalysators über eine mit feinkörnigem $SiO_2$ beschickte Absorptionskolonne geleitet. Nach Aufnahme einer Katalysatormenge, die 3,85 g $FeCl_3$/100 g $SiO_2$ entsprach, durch das Adsorptionsmittel war letzteres gesättigt. Durch Eluieren des Adsorptionsmittel mit heißem 1,2-Dichlorethan konnten 2,3 g $FeCl_3$/100 g $SiO_2$ zurückgewonnen werden. Die konzentrierte Katalysatorlösung wurde in den Chlorierungsreaktor zurückgeführt und damit die notwendige Katalysatorkonzentration im Reaktor ohne weitere Zugabe von $FeCl_3$ und $NH_3$ aufrechterhalten.

HOECHST AKTIENGESELLSCHAFT                    HOE 82/H 047

Verfahren zur Herstellung von 1,2-Dichlorethan

Patentansprüche

1. Verfahren zur Herstellung von 1,2-Dichlorethan durch
   Reaktion von Ethylen mit Chlor in einem Lösungsmittel
   in Gegenwart eines Katalysatorgemisches bestehend aus
   wasserfreiem Eisen-III-chlorid und einer Stickstoffbase bzw. einem Salz dieser Base sowie gegebenenfalls
   eines Inhibitors zur Verhinderung der Nebenproduktbildung bei einer Temperatur unterhalb des Siedepunktes
   des 1,2-Dichlorethans von etwa 20 - 100°C und Normal-
   oder Überdruck und Abtrennen des 1,2-Dichlorethans
   aus dem Chlorierungsgemisch, <u>dadurch gekennzeichnet</u>,
   daß man

   a) das Chlorierungsgemisch in eine Destillationskolonne
      fördert, aus dem Gemisch das 1,2-Dichlorethan bis
      zur Ausscheidung des Katalysators aus dem flüssigen
      Sumpfprodukt abdestilliert, aus dem Sumpfprodukt den
      ausgeschiedenen Katalysator abtrennt und letzteren
      erneut zur Umsetzung von Ethylen mit Chlor verwendet
      oder daß man

   b) das Chlorierungsgemisch über ein Adsorptionsmittel
      leitet, den im Gemisch enthaltenen Katalysator an
      dem Adsorptionsmittel adsorbiert, aus dem anfallenden Filtrat 1,2-Dichlorethan durch Destillation abtrennt und den Katalysator aus dem Adsorptionsmittel
      durch Eluierung mit einem Lösungsmittel zurückgewinnt
      oder daß man

c) entsprechend a) verfährt, jedoch bei geringen Katalysatorgehalten das katalysatorhaltige Sumpfprodukt verwirft.

2. Verfahren nach Anspruch 1, dadurch gekennzeichnet, daß die Konzentration des Eisen-III-chlorids im Reaktionsansatz 0,001 bis etwa 0,5 Gew%, insbesondere 0,002 bis 0,01 Gew%, bezogen auf die Menge des Lösungsmittels, beträgt.

3. Verfahren nach Anspruch 1 oder 2, dadurch gekennzeichnet, daß in den Reaktionsansatz eine der Eisen-III-chlorid-Konzentration äquivalente Menge der Stickstoffbase bzw. des Salzes dieser Base eingebracht wird.

4. Verfahren nach Anspruch 1 - 3, dadurch gekennzeichnet, daß das Lösungsmittel 1,2-Dichlorethan ist.

5. Verfahren nach Anspruch 1 - 4, dadurch gekennzeichnet, daß das Adsorptionsmittel Aktivkohle oder ein basischer Ionenaustauscher oder $SiO_2$ bzw. Aluminiumoxid ist.

6. Verfahren nach Anspruch 1 - 5, dadurch gekennzeichnet, daß man den Katalysator aus dem Adsorptionsmittel mit 1,2-Dichlorethan oder anderen leichtflüchtigen chlorierten Kohlenwasserstoffen oder Salzsäure eluiert.

7. Verfahren nach Anspruch 1 - 6, dadurch gekennzeichnet, daß man das katalysatorhaltige flüssige Sumpfprodukt bei einem Katalysatorgehalt von höchstens etwa 0,001 Gew% Katalysator, bezogen auf die flüssigen Anteile des Sumpfproduktes, verwirft.

| Europäisches Patentamt | **EUROPÄISCHER RECHERCHENBERICHT** | 0112544 Nummer der Anmeldung |
|---|---|---|
| | | EP 83 11 2745 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. $^3$) |
|---|---|---|---|
| P,D Y | DE-A-3 148 450 (HOECHST) <br><br> * Patentansprüche * <br><br> --- | 1-4 | C 07 C 19/045 <br> C 07 C 17/02 <br> C 07 C 17/38 |
| Y | DE-B-1 768 367 (WACKER-CHEMIE) <br> * Patentansprüche * <br><br> --- | 1,4 | |
| Y | DE-A-2 014 168 (CONTINENTAL OIL COMPANY) <br> * Patentansprüche * <br><br> --- | 1,5 | |
| Y | DE-A-1 939 391 (THE B.F. GOODRICH COMPANY) <br> * Patentansprüche * <br><br> ----- | 1,5 | |
| | | | **RECHERCHIERTE SACHGEBIETE (Int. Cl. $^3$)** <br><br> C 07 C 19/00 <br> C 07 C 17/00 |

Der vorliegende Recherchenbericht wurde für alle Patentansprüche erstellt.

| *Recherchenort* <br> DEN HAAG | *Abschlußdatum der Recherche* <br> 09-03-1984 | *Prüfer* <br> VAN GEYT J.J.A. |
|---|---|---|

EPA Form 1503 03 82